# EUROPEAN PATENT APPLICATION

(11) **EP 2 634 716 A1**
(43) Date of publication of application: **04.09.2013**
(21) Application number: 12160755.0
(22) Date of filing: 22.03.2012
(51) Int. Cl.: G06F 19/22

(54) **Tamper-proof genetic sequence processing**

(30) Priority: 28.02.2012 US 201261604007 P
(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

A genetic sequencing read is secured by computing a base string hash value comprising a hash value computed for a text string representing the base sequence of the genetic sequencing read, and generating a text representation of the genetic sequencing read comprising (i) a base string security field comprising a text representation of the computed base string hash value and (ii) the text string representing the base sequence of the genetic sequencing read. The base string hash value may be encrypted using the private key of a public key encryption system before adding to the base string security field. Read verification includes computing a test base string hash value comprising a hash value computed for the text string representing a genetic sequencing read, and comparing against the base string hash value in the base string security field of the secured genetic sequencing read.

## Description

The following relates to the genetic analysis arts, patient privacy arts, medical data integrity arts, and to applications of same such as the medical arts including oncology arts, veterinary arts, electronic patient record system arts, and so forth.

Genetic sequencing data are typically stored in a text format, such as FASTA or FASTQ. In the FASTA format, occurrences of the bases adenine (A), cytosine (C), guanine (G), and thymine (T) are represented by ASCII letters "A", "C", "G", and "T", respectively. For RNA the base thymine (T) is replaced by the base uracil (U). FASTA does not store quality score information.

The FASTQ format is an extension of FASTA that incorporates quality scores. A FASTQ representation of a read is typically in ASCII format and includes four lines: a header line as the sequence identifier; a sequence line listing the ordered sequence of bases represented by the appropriate ASCII letters "A", "C", "G", and "T" (for DNA) or "U" (for RNA); a separator line typically consisting solely of a plus ("+") sign; and a base quality scores line listing the base quality scores using suitable encoding (for example, mapping phred scores 0-93 to ASCII codes 33-126 so as to avoid numerous "unprintable" ASCII characters in the 0-32 range).

Variant formats also exist, such as QSEQ, which places all information on a single text line with tab delimiters. As another illustrative variant, some FASTQ formats extend the "+" sign separator line to include other information such as the sequence identifier, so as to mirror the header. The use of a text format such as FASTA or FASTQ makes it convenient to use and convenient to parse, and ensures platform independence and prevents loss of patient data during migration from one system to another.

However, the easy readability of text formats also raises concerns about data integrity. The genetic sequence can be compromised either accidentally (e.g., by a computer glitch or program bug altering one or more base values or quality values) or intentionally (e.g., by a malicious person altering base and/or quality values). An unexpected change in sequence data can have high impact. A change of a few bases in a chromosomal coordinate that contains a mutation for cancer or another genetic disorder can lead to a false diagnosis and/or an incorrect variant call. This can adversely affect any medical decision that relies on the data. In other areas, such as criminal forensics, tamper-proof genetic sequencing data is also desired.

One way to protect sequencing data is to encrypt it. The FASTA or FASTQ sequencing data file can be encrypted, thus preventing malicious modification of the stored encrypted data. This approach also may prevent accidental data modification, e.g. by computer glitches, since the accidental modification is likely to appear as a corruption of the encrypted data file. However, encryption makes the data less accessible, and moreover encryption of the stored FASTA or FASTQ file cannot prevent accidental (or, in some cases, even malicious) sequence modification during sequence processing. This is because the sequence must be decrypted in order to be available for processing. Other possible approaches include comparing the sequencing data undergoing processing against a reference copy of the sequence that is maintained in a highly secure fashion. However, this reduces data portability since secure sequence processing can only be performed when the reference copy can be securely referenced.

The following contemplates improved apparatuses and methods that overcome the aforementioned limitations and others.

According to one aspect, an apparatus comprises an electronic data processing device configured to perform a read securing method including: computing a base string hash value comprising a hash value computed for a text string representing the base sequence of the genetic sequencing read; and generating a text representation of the genetic sequencing read comprising (i) a base string security field comprising a text representation of the computed base string hash value and (ii) the text string representing the base sequence of the genetic sequencing read. In some embodiments the read securing method further comprises encrypting the computed base string hash value using the private key of a public key encryption system, wherein the base string security field comprises a text representation of the encrypted computed base string hash value. In some embodiments the apparatus further includes an electronic data processing device configured to perform a read verification method including: computing a test base string hash value comprising a hash value computed for the text string representing the base sequence of the genetic sequencing read; and verifying the text representation of the genetic sequencing read by comparing the test base string hash value against the text representation of the computed base string hash value stored in the base string security field. In such embodiments, the read securing method and the read verification method may both be performed by the same electronic data processing device; or, the read securing method and the read verification method may be performed by different electronic data processing devices.

According to another aspect, a method is disclosed. A text representation is received of a genetic sequencing read comprising (i) a base string security field containing a text representation of a reference base string hash value computed using a hash function and (ii) a base string representing a sequence of bases. A test base string hash value is computed for the base string using the hash function. The text representation of the genetic sequencing read is verified by comparing the test base string hash value against the reference base string hash value. These operations are suitably performed by an electronic data processing device. In some embodiments the received text representation of the genetic sequencing read further comprises (iii) a quality string security field containing a text representation of a reference quality string hash value computed using the hash function and (iv) a quality string representing base quality values for the sequence of bases, and the method further comprises computing a test quality string hash value for the quality string using the hash function, and the verifying further includes comparing the test quality string hash value against the reference quality string hash value.

According to another aspect, a non-transitory storage medium stores instructions executable by one or more electronic data processing devices to perform a method as set forth in the immediately preceding paragraph.

One advantage resides in ensuring the integrity of patient genetic sequencing data while maintaining the convenience of text formatting of the sequencing data.

Another advantage resides in detecting accidental modification of sequencing data.

Another advantage resides in detecting malicious modification of sequencing data.

Another advantage resides in achieving the foregoing benefits while retaining the use of the existing FASTA, FASTQ, or other textual sequencing data format.

Numerous additional advantages and benefits will become apparent to those of ordinary skill in the art upon reading the following detailed description.

The invention may take form in various components and arrangements of components, and in various process operations and arrangements of process operations. The drawings are only for the purpose of illustrating preferred embodiments and are not to be construed as limiting the invention.
- FIGURE 1: diagrammatically shows a genetic analysis system that stores and processes genetic sequencing data in a portable and accessible text format while ensuring the integrity of the sequencing data is maintained.
- FIGURE 2: diagrammatically shows the security code tagger module of the system of FIGURE 1.
- FIGURE 3: diagrammatically shows the validation module of the system of FIGURE 1.

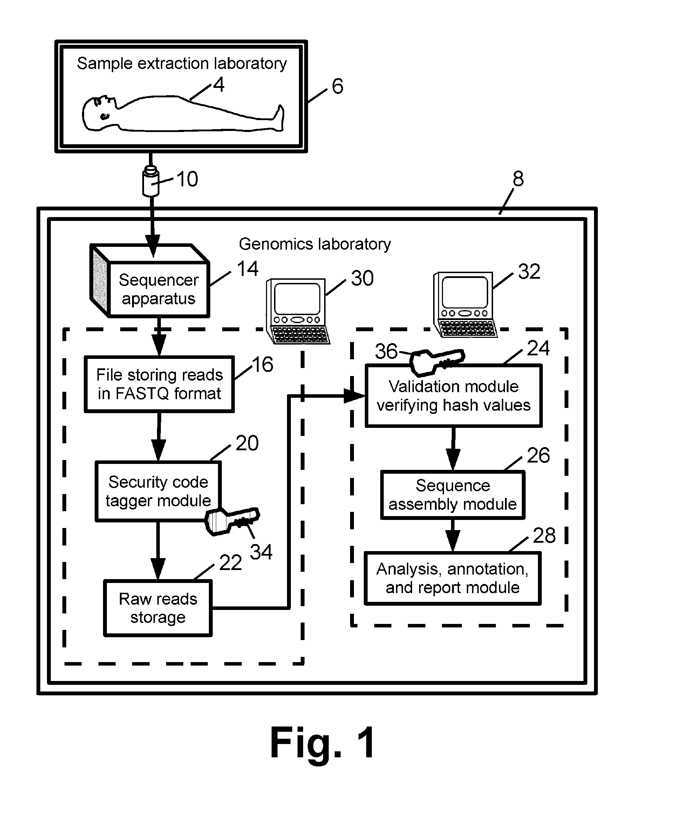

With reference to FIGURE 1, an illustrative clinical or diagnostic application is described. A patient **4** undergoes tissue sample extraction in a sample extraction laboratory **6** to generate a tissue sample that is processed by a genomics laboratory **8** to sequence and analyze DNA and/or RNA of the sample. For example, the sampling laboratory **6** may extract from the subject **4** a tissue sample from a malignant lesion, optionally along with a normal tissue sample extracted from elsewhere in the patient **4.** As some other illustrative examples, the sample extraction may comprise a biopsy procedure employing a biopsy needle or other interventional instrument, the plucking of a hair sample to obtain the follicle containing DNA, the drawing of a blood using a hypodermic needle, or so forth. FIGURE 1 shows an illustrative extracted tissue sample **10.** It is noted that while the illustrative embodiment is operative on patient **4** in a clinical application, in other applications the tissue sample may be extracted from a nonhuman subject such as a veterinary subject, a biology research subject, or so forth. The application of the genetic analysis can be varied, such as: medical or veterinary diagnosis, monitoring, or the like; generating a genetic database for anthropology studies (e.g., population migration studies, et cetera); subject assessment in a clinical or preclinical study; or so forth. It will also be noted that in illustrative FIGURE 1 the sample **10** is represented by an illustrative vial; however, it is to be understood that the sample **10** may in general take any form suitable for the type of tissue that has been sampled, and may be contained or supported by any suitable container or support for that type of tissue. For example, the sample **10** may be a fluid sample, a surface sample (e.g. obtained by oral swabs and disposed on a sterile slide or other suitable surface), or so forth.

At the genomics laboratory **8,** the tissue sample **10** is processed by a sequencer apparatus **14** to generate sequencing reads. The sequencer apparatus **14** may be a next generation sequencing (NGS) apparatus or a more conventional sequencing apparatus such as a Sanger sequencing facility. The sequencer apparatus **14** may in some embodiments be a commercial sequencing apparatus such as are available from Illumina, San Diego, CA, USA; Knome, Cambridge, MA, USA; Ion Torrent Inc., Guilford, CT, USA; or other NGS system vendors; however, a noncommercial or custom-built sequencer is also contemplated. A portion of the functionality of the sequencer apparatus may also be performed manually. The generated sequencing reads are optionally filtered to remove duplicate reads and/or to discard reads having base quality (e.g. phred or phred-like) scores below 20 (or below another selected base quality score threshold). The remaining sequencing reads are stored in a file **16** that stores the reads in a FASTQ format (as shown) or in another text format (e.g., FASTA or Qseq). In embodiments in which the sequencer apparatus **14** is an automated commercial product, the sequencer apparatus **14** is suitably operated in its usual and ordinary manner and outputs the sequencing reads **16** formatted in a standard output of the sequencer apparatus **14.**

With continuing reference to FIGURE 1, a security code tagger module **20** annotates the file **16** with security hash values that are associated with strings of bases and optionally also with corresponding quality strings. A "hash value" as used herein is a value computed from a sequence of arbitrary length and content that is useful for detecting an error in the sequence. Typically, the hash value is computed by applying a hash function (also sometimes called a checksum function) to the string of bases or to the quality string. Some suitable hash functions include the MD5 hash function, the SHA-2 family of hash functions, or so forth. The annotations are added to the FASTA or FASTQ file as an additional metadata field stored in the header line (and/or optionally as an added field stored in the "+" separator line of the FASTQ format). The hash function is chosen such that, for a string of bases or quality values, the computed has value is highly likely to change if even one base or quality value of the string is changed. The resulting FASTA, FASTQ, or other text file storing the reads and the annotated hash values is stored in a raw reads storage **22.**

With continuing reference to FIGURE 1, when the sequencing data are to be processed, a validation module **24** retrieves the raw reads file, computes the hash values for the base and quality strings, and compares the computed hash values with those annotated in the header of the file. If the computed and annotated hash values do not match, then it is assumed that the sequencing data has been corrupted (either accidentally or maliciously) and remedial action is taken.

On the other hand, if the computed and annotated hash values match, then sequencing data processing is performed. For example, in illustrative FIGURE 1 the (validated) raw reads are assembled by a sequence assembly module **26** to generate a genetic sequence for the tissue sample. The assembly performed by the assembly module **26** can be *de novo* alignment of overlapping portions sequencing reads, or can be mapping of the sequencing reads to a reference sequence while allowing for a certain fraction (e.g., 5-10%) of base mismatches. In the latter case the reference sequence can be, for example, a standard reference sequence of the human genome in the illustrative case of human patient **4.** The resulting aligned sequence dataset can be variously stored, compared with other sequences, analyzed to detect probative variants, or so forth. In the illustrative example, an analysis, annotation, and report module **28** compares the aligned sequence with an annotated reference sequence in order to identify variants that correlate with a disease (e.g., a type of cancer) or other information.

The various processing components **20, 24, 26, 28** are suitably embodied by one or more computers or other electronic data processing devices **30, 32.** By way of illustration, the electronic data processing device or devices **30, 32** may include: a notebook computer; a desktop computer; a mobile device such as a smartphone, tablet computer, personal data assistant (PDA), or the like; a network server computer accessible via the Internet and/or a local wired/wireless data network; various combinations thereof; or so forth. The raw reads storage **22** is suitably embodied as random access memory (RAM), flash memory, or other type of electronic storage, or as a hard disk or other type of magnetic storage, or so forth. In some embodiments, a single computer performs all of the various processing components **20, 24, 26, 28.** However, in the illustrative example, a first computer **30** embodies the "acquisition" component **20** (and is optionally integrated with the sequencer apparatus **14),** while a second computer **32** embodies the validation and genetic sequence processing components **24, 26, 28.** This is a practical arrangement if, for example, the processing components **24, 26, 28** are purchased separately from the sequence acquisition system **14, 20,** or if the processing components **24, 26, 28** are custom-built. In embodiments employing the separate computers (or other electronic data processing devices) **30, 32,** the security code tagger module **20** executing on the first computer **30** and the validation module **24** executing on the second computer **32** should be synchronized in that both modules **20, 24** should use the same hash function (and the same optional preprocessing, e.g. whitespace removal) for generating the hash values. For example, in one contemplated approach the manufacturer of the sequencing system **14, 20** includes the security code tagger module **20** as a built-in feature of the sequencing system **14, 20.** In this example, the manufacturer of the sequencing system **14, 20** would then provide the validation module **24** as an add-on or "plug-in" package for the processing system **26, 28.**

The disclosed techniques for processing raw reads in memory-efficient and computationally-efficient ways are also suitably embodied as a non-transitory storage medium storing instructions executable by the illustrative computer or other electronic data processing device **30, 32** to perform the disclosed reads processing. The non-transitory storage medium storing the executable instructions may, for example, include: a hard disk drive or other magnetic storage medium; an optical disk or other optical storage medium; a flash memory, random access memory (RAM), read-only memory (ROM), or other electronic storage medium; or so forth.

With continuing reference to FIGURE 1, the foregoing approach of computing hash values for base strings (and optionally for quality strings) and annotating the hash values to the text file representing the read is sufficient to detect inadvertent modification of the read due to a computer glitch, software bug, or unintentional human error. However, this approach may not be sufficient to detect malicious modification of the read. This is because it would be relatively straightforward for a skilled artisan to modify the read and then re-compute the hash value for the modified read and update the hash value annotation of the file so that the annotated hash value matches the maliciously altered base sequence. To prevent malicious modification of the base sequences, in some embodiments the security code tagger module **20** encrypts the hash values with a private key **34** of a public key encryption system, and annotates the text file with the encrypted hash values. The validation module **24** then uses a public key **36** of the public key encryption system to decrypt the annotated (and encrypted) hash values prior to comparing the values with the hash values computed at the validation module **24.** This approach is based on the following property of public key encryption systems: the public key and the private key are not interchangeable. Thus, as long as the private key **34** is kept secure, a malicious person could modify the base sequence and could generate the correct hash value -- but, the malicious person could not encrypt that hash value because the private key is unavailable.

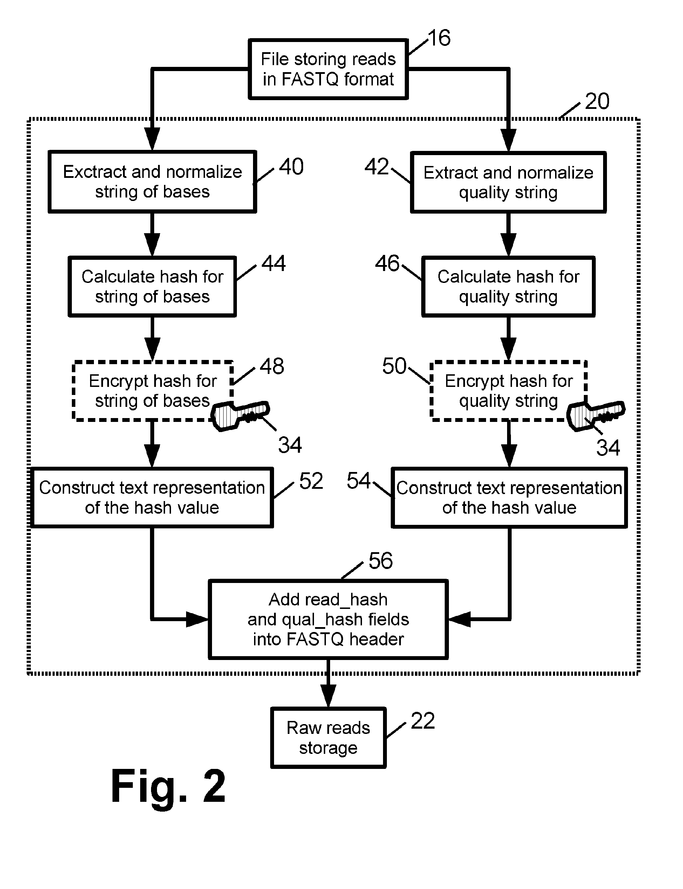

With continuing reference to FIGURE 1 and with further reference to FIGURE 2, a read securing method suitably performed by the illustrative security code tagger module **20** is described. The file **16** storing the read is assumed to be in a FASTQ format. In an operation **40,** a base string is generated by extracting and normalizing the sequence of bases from the FASTQ formatted text representation of the genetic sequencing read. The operation **40** optionally includes various normalization or standardization operations, such as stripping "whitespace" characters (e.g., spaces, tabs, carriage return or linefeed "special characters", or so forth) from the base string, converting the base symbols from lowercase to uppercase if needed, or so forth. In an operation **42,** the quality string comprising base quality scores for the bases of the base string is similarly extracted and normalized.

In an operation **44,** a suitable hash function is applied to the (normalized) base string to generate a reference base string hash value. The hash function may, by way of illustrative example, be an MD5 hash function, a hash function belonging to the SHA-2 family of hash functions, or so forth. More generally, the hash function may be any function that maps the (normalized) base string to a more compact value that can be represented as a text representation. While MD5 or SHA-2 hash functions have advantages in terms of compactness and providing low collision rates, the hash function can alternatively be a relatively simple function if potentially more frequent collisions are not an issue. (Note that a collision will only arise if a modified base string happens to produce the same hash value as the original, unmodified base string. If base string modification events are rare, then the likelihood of such an occurrence should be low even for a hash function with a relatively high collision rate). In an operation **46,** a suitable hash function is applied to the (normalized) quality string to generate a reference quality string hash value. The same hash function is typically used in both hashing operations **44, 46,** although the use of different hash functions is also contemplated.

As noted previously, the hash values, by themselves, cannot secure against malicious modification of the genetic sequencing read, because the malicious person can update the hash value to match the modified read. Accordingly, in embodiments that are intended to be secure against malicious modifications, an encryption operation **48** is performed using the private key **34** of the public key encryption system in order to encrypt the reference base string hash value. Similarly, an encryption operation **50** uses the private key **34** to encrypt the text representation of the reference quality string hash value. (Operations **48, 50** are optionally omitted if security against malicious modification is not desired; hence encryption operations **48, 50** are shown in phantom in FIGURE 2).

The output of a typical hash function is not text. For example, the MD5 hash function outputs a 128 bit binary value, while various members of the SHA-2 family of hash functions output binary values of 224 bits, 256 bits, 512 bits, or so forth. The public key encryption performed by optional operations **48, 50** typically does not change the number of bits. For example, public key encryption of a 128 bit hash value generated by the MD5 hash function will produce an encrypted 128 bit hash value. Regardless of whether the hash values are encrypted, however, the resulting (encrypted or unencrypted) hash values are not text.

Accordingly, in an operation **52** a text representation of the (optionally encrypted) reference base string hash value is constructed. In a suitable approach, the text representation of the (optionally encrypted) reference base string hash value is a constructed as a corresponding hexadecimal value whose "digits" are represented by appropriate ASCII characters, e.g. in the range "0"..."9" and "A"..."F". Thus, by way of illustrative example, the 128 bit hash value generated by the MD5 hash (and optionally encrypted) corresponds to 32 hexadecimal "digits", and so the text representation of the (optionally encrypted) reference base string hash value is a 32 character string made up of characters from the sets {"0"..."9"} and {"A"..."F"). The text representation can optionally be in a uucode/base64 format to provide a more compact text representation. Other text representations are also contemplated, such as converting the hash value to a decimal value represented as an ASCII string. An analogous operation **54** constructs a text representation of the reference quality string hash value.

In an operation **56,** the text representation of the (optionally encrypted) reference base string hash value is added to the header of the FASTQ text representation of the genetic sequencing read as a suitably formatted base string security field. For example, the base string security field can have the format "read_hash=################################" where "read_hash=" is an identifying prefix and "################################" is a thirty-two character sequence of hexadecimal digits representing the (optionally encrypted) reference base string hash value. The operation **58** also suitably adds a quality string security field containing the text representation of the reference quality string hash value with a suitable prefix such as "qual_hash=". The resulting modified FASTQ formatted text representation of the genetic sequencing read, including the base string security field and the quality string security field in the FASTQ header, is stored in the raw reads storage **22**.

By way of illustrative example, the following is an illustrative example of a file including two genetic sequencing reads in the FASTQ format that each include an annotated base string security field (prefixed by "read_md5:") and an annotated quality string security field (prefixed by "qual_md5:"). As suggested by the prefix, this illustrative example employs the MD5 hash function.
@V15-1345:1125/1 read_md5: fe5d2ab546be9d1707e96066d3713326 qual_md5: 7a2754ff62c8f272f9675ec91432b592
ATCAGGACCACGTTCAGGTACCTGTTCTTGTTGGGGCTGCAGTCGTAGGCCA CCTTCTCCTCGTTCAGCGCCTTCTCCTCGGCCTCCCGC
+
BS\ceeeeggfggiiiihgghihifiihhhgiihhhfhighigihgffd]bd_efc_\Zb^'Y^a^X^BBBBBBBB BBBBBBBBBBBBBB
@V15-1345:1603/1 read_md5: 2c489d8209ee75b1f0821cf9e5943d1d qual_md5: ad88952ede8b50e53e0145657ee12857
CCCATTTCTGCTCTGTCTCAATCACGAAATCACAGACAATGCACAAGATATG CGAGAGAAGACTCACATTGTACACCACTGTTATCCAAG
+
__^ceeeeggegghhdbfhfhfdd_dcgghhfhffeg]^XaI^X^caXcfgfd[cZU_\b_'fg_\''^^'de_'b^ \^_bZ'Y]'b_b_

It will be appreciated that the FASTQ file can include as few as a single genetic sequencing read, or two reads (as in the above example) or higher number of reads.

In embodiments that do not retain base quality values (e.g., the FASTA format), the operations **42, 46, 50, 54** are suitably omitted. The FASTA file equivalent for the above example would be as follows:
@V15-1345:1125/1 md5: fe5d2ab546be9d1707e96066d3713326
   ATCAGGACCACGTTCAGGTACCTGTTCTTGTTGGGGCTGCAGTCGTAGGCCA CCTTCTCCTCGTTCAGCGCCTTCTCCTCGGCCTCCCGC
@V15-1345:1603/1 md5: 2c489d8209ee75b1f0821cf9e5943d1d
   CCCATTTCTGCTCTGTCTCAATCACGAAATCACAGACAATGCACAAGATATG CGAGAGAAGACTCACATTGTACACCACTGTTATCCAAG

In this example since there are only read strings, but not quality strings, the prefix of the base string security field is shortened to "md5:". In these examples the hexadecimal "digits" for numeral values greater than 9 are represented by lower-case letter, which can be convenient if the base strings representing sequences of bases employ upper-case letters "A", "C", "G", and "T" (for DNA) or "U" (for RNA). However, upper-case letters "A"..."F" can also be used to represent the hexadecimal "digits".

While the FASTA or FASTQ header represents a natural place to store the base string security field (and the optional quality string security field), other locations for this information are also contemplated, such as in the "+" separator line of the FASTQ format. For example, in one variant approach, the base string security field is stored in the FASTQ header while the quality string security field is stored as part of the "+" separator line. The base string security field (and the optional quality string security field) can also be incorporated as part of the read name (or as part of a file name).

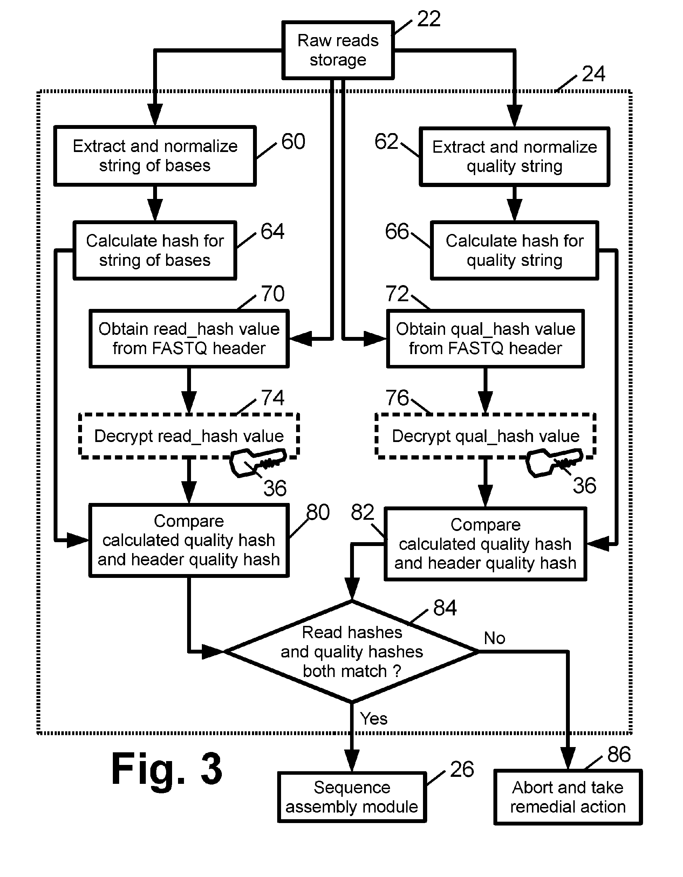

With continuing reference to FIGURE 1 and with further reference to FIGURE 3, a read verification method suitably performed by the illustrative validation module **24** on a read stored in the raw reads storage **22** is described. The genetic sequencing read is, in this example, assumed to be stored in a FASTQ format with the base string security field and the quality string security field added to the FASTQ header as described with reference to FIGURE 2. In an operation **60,** a base string is generated by extracting and normalizing the sequence of bases from the FASTQ formatted text representation of the genetic sequencing read. The operation **60** includes the same (optional) normalization or standardization operations as in the operation **40** performed by the security code tagger module **20.** In an operation **62,** the quality string comprising base quality scores for the bases of the base string is similarly extracted and normalized, using a procedure paralleling the operation **42** performed by the security code tagger module **20.**

In an operation **64,** the same hash function as was applied in the operation **44** performed by the security code tagger module **20** is applied to the (normalized) base string generated by the operation **60** in order to generate a test base string hash value. In an operation **66,** the hash function as was applied in operation **46** performed by the security code tagger module **20** is applied to the (normalized) quality string to generate a test quality string hash value.

These test hash values are to be compared with the reference hash values stored in the base and quality string security fields of the FASTQ header. Accordingly, in an operation **70** the reference base string hash value is extracted from the base string security field (e.g., as identified by the prefix "read_hash=" in the illustrative example). Similarly, in an operation **72** the reference quality string hash value is extracted from the quality string security field (e.g., as identified by the prefix "quality_hash=" in the illustrative example). The operations **70, 72** may optionally perform processing of the stored value to convert it to a desired format. For example, if the reference hash values are stored as "################################" (i.e., a thirty-two character sequence of hexadecimal digits representing the optionally encrypted reference hash value) then the processing may convert this text string to a numerical value (suitably represented in binary, decimal, or another numeric representation). If the reference hash values were encrypted by the security code tagger module **20,** then in an operation **74** the reference base string hash value is decrypted using the public key **36** of the public key encryption system. Since the public key is different from the private key used for encryption, and since the private key cannot be derived from the public key in practice, it follows that the public key **36** can be freely distributed for use in reads verification. In a similar operation **76** the reference quality string hash value is decrypted using the public key **36** of the public key encryption system.

In an operation **80,** the test base string hash value output by the operation **64** is compared with the reference base string hash value output by the operation **70** (after decryption as per optional decryption operation **74).** In an operation **82,** the test quality string hash value output by the operation **66** is compared with the reference quality string hash value output by the operation **72** (after decryption as per optional decryption operation **76).** The comparison operations **80, 82** can perform various optional pre-processing in order to ensure the test and reference hash values are in comparable (i.e., the same) format. In embodiments in which the reference hash values are encrypted, the text string representing the hash value typically is converted to a numeric (e.g., binary) value that is then decrypted - accordingly, the comparison operations **80, 82** suitably compare numeric values. On the other hand, if no encryption is employed, then an alternative approach is to convert the test hash values to text strings of the same format in which the reference hash values are stored in the FASTQ file, and the comparisons **80, 82** can compare text strings representing the respective test and reference hash values.

In an operation **86,** the results of the comparisons **80, 82** are used to verify the integrity of the genetic sequencing read. If the comparison **80** indicates that the test and reference base hash values are identical and the comparison **82** indicates that the test and reference quality hash values are identical, then the genetic sequencing read is considered to be verified, and downstream processing may commence (e.g., in the illustrative example the verified read is passed to the sequence assembly module **26** which performs a sequence assembly process combining the genetic sequencing read (as represented by the verified text representation of the genetic sequencing read) with other reads to generate an aligned genetic sequence. On the other hand, if either (1) the comparison **80** indicates that the test and reference base hash values are not identical or (2) the comparison **82** indicates that the test and reference quality hash values are not identical, then the verification fails and process flow passes to an abort operation **86** which prevents the sequencing read from being used, and optionally takes remedial action such as (by way of illustrative example) displaying a warning to the user on a display device (e.g., LCD display) informing the user that the genetic sequencing read has failed verification.

In embodiments that do not retain base quality values (e.g., the FASTA format), the operations **62, 66, 72, 76,** and **82** are suitably omitted, and the decision operation **86** is based on the output of the comparison **80** alone. Moreover, as noted previously the base string security field (and the optional quality string security field) may be located elsewhere than in the header, such as in the "+" separator line of the FASTQ format or as part of the read name (or as part of a file name).

It is also noteworthy that the validation performed by the validation module **24** can be skipped entirely, even if the genetic sequencing read has been annotated by the security code tagger module **20** to include the base string and quality string security fields. For example, some genetic sequence processing systems may not include the validation module **24** or equivalent validation functionality. In such cases, the text representation of the genetic sequencing read is still in standard FASTQ format, albeit with the additional base string and quality string security fields), and so the genetic sequencing read as per conventional processing of FASTQ reads data. This ensures compatibility with "legacy" genetic sequence processing systems that do not support the reads verification techniques disclosed herein.

In the embodiments of FIGURES 2 and 3, malicious alteration can be addressed by encrypting the reference base string and quality string hash values. Another approach is to leave these hash values unencrypted, and instead compute a hash value for the entire read file. In the previous FASTQ example the result would be as follows:
@V15-1345:1125/1 read_md5: fe5d2ab546be9d1707e96066d3713326 qual_md5: 7a2754ff62c8f272f9675ec91432b592
   encrypted_file_md5:e1f7bcac702cacde934f0796d8a137d2
   ATCAGGACCACGTTCAGGTACCTGTTCTTGTTGGGGCTGCAGTCGTAGGCCA CCTTCTCCTCGTTCAGCGCCTTCTCCTCGGCCTCCCGC
   +
   BS\ceeeeggfggiiiihgghihifiihhhgiihhhfhighigihgffd]bd_efc_\Zb^Y^a^X^BBBBBBBB BBBBBBBBBBBBBB
@V15-1345:1603/1 read_md5: 2c489d8209ee75b1f0821cf9e5943d1d qual_md5:
   ad88952ede8b50e53e0145657ee12857
   CCCATTTCTGCTCTGTCTCAATCACGAAATCACAGACAATGCACAAGATATG CGAGAGAAGACTCACATTGTACACCACTGTTATCCAAG
   +
   __^ceeeeggegghhdbfhfhfdd_dcgghhfhffeg]^XaI^X^caXcfgfd[cZU_\b_'fg_\''^^'de_'b^ \^_bZ'Y]'b_b_

In this case a file security field prefixed by "encrypted_file_md5:" stores a reference hash value for the entire file. In a suitable approach, the security code tagger module **20** computes and encrypts the reference hash value for the entire text file (e.g., FASTQ file) and then adds the file security field containing the encrypted reference hash value for the file to the header of the first genetic sequencing read of the file. The validation module **24** then extracts the encrypted reference hash value for the entire file and decrypts it to recover the reference hash value, then strips off the file security field, computes the test hash value for the entire file, and compares test hash value with the reference hash file. In this processing sequence the validation module must strip the file security field off the file before computing the hash value for the file because the reference hash value for the file was computed prior to adding the file security field. An advantage of this approach is that it secures the entire text file, not just the read strings and quality strings. Thus, even a modification of the FASTQ header would be detected.

The disclosed security approaches increase the file size by the amount needed to store the security fields. As FASTQ data can encode several million sequence fragments (i.e. several million genetic sequencing reads), storing reference base string and quality string hash values for each fragment (i.e. read) entails substantial storage space. An alternative is to store only the first few characters of each hash value string representation. For example, rather than storing the entire 32 hexadecimal digits representing a 128 bit MD5 hash value, only the first 5 digits (or 10 digits, et cetera) can be stored. This truncation reduces the storage requirements, while still being reasonably tamper proof if the hash values are generated by the MD5 hash function or another hash function having well distributed hash values. It is also contemplated to generate and store only the base string hash value (either full-length or truncated) but not the quality string hash value. Yet another approach for balancing memory usage and security is to compute/store hash values only for a certain randomly selected fraction of the reads, e.g. 5% of the reads. This provides "spot-checking" of the integrity of the reads.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof

## Claims

1. An apparatus comprising:
an electronic data processing device **(30)** configured to perform a read securing method including:
computing a base string hash value comprising a hash value computed for a text string representing the base sequence of the genetic sequencing read; and
generating a text representation of the genetic sequencing read comprising (i) a base string security field comprising a text representation of the computed base string hash value and (ii) the text string representing the base sequence of the genetic sequencing read.

2. The apparatus of claim **1,** wherein the generating comprises:
generating a FASTA or FASTQ representation of the genetic sequencing read comprising (i) a header including the base string security field comprising the text representation of the computed base string hash value and (ii) the text string representing the base sequence of the genetic sequencing read.

3. The apparatus of claim **2,** wherein the read securing method performed by the electronic data processing device **(30)** further includes:
receiving a FASTA or FASTQ representation of the genetic sequencing read;
wherein the generating comprises adding the base string security field to the header of the received FASTA or FASTQ representation of the genetic sequencing read.

4. The apparatus of any one of claims **1-3,** wherein the read securing method performed by the electronic data processing device **(30)** further includes:
computing a quality string hash value comprising a hash value computed for a quality string representing base quality scores for the base sequence of the genetic sequencing read;
wherein the generating includes generating the text representation of the genetic sequencing read comprising (i) the base string security field, (ii) a quality string security field comprising a text representation of the computed quality string hash value, (iii) the text string representing the base sequence of the genetic sequencing read, and (iv) the quality string representing base quality scores for the base sequence of the genetic sequencing read.

5. The apparatus of claim **4,** wherein the generating comprises:
generating a FASTQ representation of the genetic sequencing read comprising a header including the base string security field and the quality string security field.

6. The apparatus of claim **5,** wherein the read securing method performed by the electronic data processing device **(30)** further includes:
receiving a FASTQ representation of the genetic sequencing read;
wherein the generating comprises adding the base string security field and the quality string security field to the header of the received FASTQ representation of the genetic sequencing read.

7. The apparatus of any one of claims **4-6,** wherein the read securing method performed by the electronic data processing device **(30)** further includes:
encrypting the computed base string hash value using the private key of a public key encryption system;
encrypting the computed quality string hash value using the private key of the public key encryption system;
wherein the base string security field comprises a text representation of the encrypted computed base string hash value; and
wherein the quality string security field comprises a text representation of the encrypted computed quality string hash value.

8. The apparatus of claim 7, further comprising:
an electronic data processing device **(32)** configured to perform a read verification method including:
decrypting the encrypted computed base string hash value using the public key of a public key encryption system to generate a reference base string hash value;
decrypting the encrypted computed quality string hash value using the public key of the public key encryption system to generate a reference quality string hash value;
computing a test base string hash value comprising a hash value computed for the text string representing the base sequence of the genetic sequencing read;
computing a test quality string hash value comprising a hash value computed for the quality string; and
verifying the text representation of the genetic sequencing read by comparing the test base string hash value against the reference base string hash value and the test quality string hash value against the reference base string hash value.

9. The apparatus of any one of claims **4-6,** further comprising:
an electronic data processing device **(32)** configured to perform a read verification method including:
computing a test base string hash value comprising a hash value computed for the text string representing the base sequence of the genetic sequencing read;
computing a test quality string hash value comprising a hash value computed for the quality string; and
verifying the text representation of the genetic sequencing read by comparing the test base string hash value against the text representation of the computed base string hash value stored in the base string security field and the test quality string hash value against the text representation of the computed quality string hash value base stored in the quality string security field.

10. The apparatus of any one of claims **1-6,** wherein the read securing method performed by the electronic data processing device **(30)** further includes:
encrypting the computed base string hash value using the private key of a public key encryption system;
wherein the base string security field comprises a text representation of the encrypted computed base string hash value.

11. The apparatus of claim **10,** further comprising:
an electronic data processing device **(32)** configured to perform a read verification method including:
decrypting the encrypted computed base string hash value using the public key of a public key encryption system to generate a reference base string hash value;
computing a test base string hash value comprising a hash value computed for the text string representing the base sequence of the genetic sequencing read; and
verifying the text representation of the genetic sequencing read by comparing the test base string hash value against the reference base string hash value.

12. The apparatus of any one of claims **1-6,** further comprising:
an electronic data processing device **(32)** configured to perform a read verification method including:
computing a test base string hash value comprising a hash value computed for the text string representing the base sequence of the genetic sequencing read; and
verifying the text representation of the genetic sequencing read by comparing the test base string hash value against the text representation of the computed base string hash value stored in the base string security field.

13. A method comprising:
receiving a text representation of a genetic sequencing read comprising (i) a base string security field containing a text representation of a reference base string hash value computed using a hash function and (ii) a base string representing a sequence of bases;
computing a test base string hash value for the base string using the hash function;
and
verifying the text representation of the genetic sequencing read by comparing the test base string hash value against the reference base string hash value;
wherein the computing and the verifying are performed by an electronic data processing device **(32).**

14. The method of claim **13,** wherein the text representation of the reference base string hash value is encrypted using the private key of a public key encryption system and the method further comprises:
decrypting the text representation of the reference base string hash value using the public key of the public key encryption system;
wherein the verifying uses the decrypted representation of the reference base string hash value; and
wherein the decrypting is also performed by the electronic data processing device **(32).**

15. A non-transitory storage medium storing instructions executable by one or more electronic data processing devices **(30, 32)** to perform a method as set forth in any one of claims **13-15.**


SEQUENCE LISTING

<110>  Koninklijke Philips Electronics N.V.

<120>  TAMPER-PROOF GENETIC SEQUENCE PROCESSING

<130>  2011PF02381

<160>  2

<170>  PatentIn version 3.5

<210>  1
<211>  90
<212>  DNA
<213>  Homo sapiens

<400>  1
atcaggacca cgttcaggta cctgttcttg ttggggctgc agtcgtaggc caccttctcc       60

tcgttcagcg ccttctcctc ggcctcccgc                                       90


<210>  2
<211>  90
<212>  DNA
<213>  Homo sapiens

<400>  2
cccatttctg ctctgtctca atcacgaaat cacagacaat gcacaagata tgcgagagaa       60

gactcacatt gtacaccact gttatccaag                                       90